# EUROPEAN PATENT APPLICATION

(11) **EP 4 725 958 A1**
(43) Date of publication of application: **15.04.2026**
(21) Application number: 24818768.4
(22) Date of filing: 07.06.2024
(51) Int. Cl.: C07K 7/62, A61K 38/08, A61K 38/12, A61P 31/04

(54) **NOVEL DECAPEPTIDE AND USE THEREOF IN ANTIBACTERIAL**

(30) Priority: 09.06.2023 CN 202310694583
(71) Applicant: Methycure Biotech Corp., Nanjing, Jiangsu 210000 (CN)
(72) Inventor: CHEN, Qihan, Nanjing, Jiangsu 211100 (CN); WANG, Qiang, Nanjing, Jiangsu 210000 (CN)
(74) Representative: Klöckner, Christoph
(86) International application number: PCT/CN2024/097969
(87) International publication number: WO 2024/251238

(57) **Abstract**

A novel decapeptide capable of inhibiting the growth of bacteria having drug resistance to existing polymyxin, a pharmaceutical composition comprising the novel decapeptide, and a use thereof in the treatment or prevention of bacterial infection.

## Description

This application claims priority to Chinese Patent Application No. 202310694583.1, filed on June 9, 2023, entitled " NOVEL DECAPEPTIDE AND USE THEREOF IN ANTIBACTERIAL", the entire contents of which are incorporated herein by reference.

### TECHNICAL FIELD

The present disclosure relates to a novel polymyxin-like compound, which can inhibit the growth of bacteria resistant to existing polymyxins. The present disclosure also relates to the use of the novel polymyxin-like compound in treating or preventing bacterial infections.

### BACKGROUND ART

Polymyxin is a polycationic antimicrobial peptide. It binds to the anionic phosphate groups in lipid A of the inner membrane of Gram-negative bacteria, causing displacement of divalent cations originally bound to the phosphate groups on lipid A of the inner membrane, thereby disrupting membrane stability. Subsequently, the hydrophobic N-terminal fatty acyl chain and the amino acids at positions 6 and 7 of polymyxin insert into the hydrophobic region of the outer membrane mainly composed of the fatty acyl chains of lipid A. The insertion of polymyxin into the phospholipid bilayer disrupts the structural integrity of the membrane and promotes the passage of other polymyxin molecules through the outer membrane. Ultimately, polymyxin traverses the interface between the hydrophilic head groups and the fatty acyl chains, disrupts the physical integrity of the inner membrane phospholipid bilayer through membrane thinning, leading to inner membrane lysis and cell death.

Polymyxins include polymyxin A-E, and the main ones on the market currently are polymyxin B and polymyxin E. Polymyxins have significant antibacterial activity against some Gram-negative bacilli such as *Escherichia coli, Klebsiella pneumoniae, Acinetobacter* species*,* and *Pseudomonas aeruginosa.* In recent years, due to the lack of other effective antimicrobial agents, polymyxins have been considered the drug of choice for treating infections caused by carbapenem-resistant *Pseudomonas aeruginosa, Klebsiella pneumoniae,* and *Acinetobacter baumannii,* and are often used as a last line of defense.

However, a growing number of studies have reported bacterial resistance to polymyxins. The resistance mechanisms of polymyxins are mainly divided into two categories: one is chromosome-mediated resistance, and the other is plasmid-mediated resistance. Chromosome-mediated resistance poses a relatively low risk of transmission, whereas the exchange of resistance gene sequences on plasmids occurs very easily, leading to very rapid spread of polymyxin resistance among bacteria, resulting in a "collapse of the last line of defense." The principles of the two resistance mechanisms are almost identical. Modification of lipopolysaccharide is achieved by partial substitution of the phosphate groups on lipid A with the cationic groups 4-amino-4-deoxy-L-arabinose (L-Ara4N) or phosphoethanolamine (PEtN), which reduces the negative charge of the outer membrane, hinders the binding of polymyxin to bacteria, and weakens the bactericidal effect of polymyxin.

### SUMMARY OF THE INVENTION

In one aspect, the present disclosure provides a compound of the following formula (I), a derivative thereof, a pharmaceutically acceptable salt thereof, or a solvate thereof:

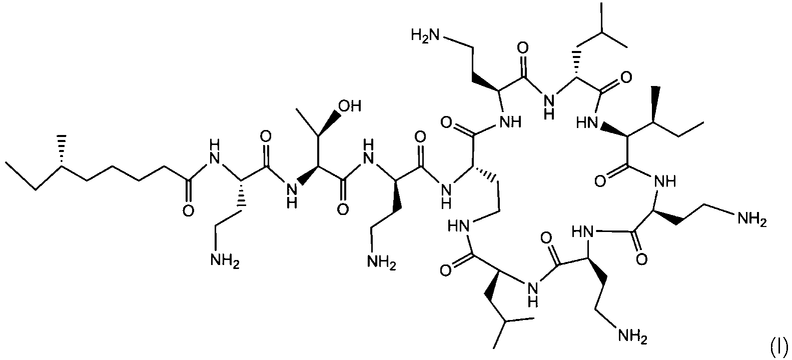

In some embodiments, the salt is selected from the group consisting of hydrochloride, sulfate, phosphate, acetate, oxalate, succinate, fumarate, maleate, lactate, malate, tartrate, citrate, picrate, and any combination thereof.

In some embodiments, the solvate is a hydrate or an ethanolate.

In some embodiments, the derivative is a PEG derivative or a polysaccharide derivative of the compound.

In another aspect, the present disclosure provides a pharmaceutical composition, comprising: 1) the compound, a derivative thereof, a pharmaceutically acceptable salt thereof, or a solvate thereof as described above; and
2) a pharmaceutically acceptable carrier.

In some embodiments, the pharmaceutical composition further comprises one or more antibiotics.

In another aspect, the present disclosure provides the use of the compound, a derivative thereof, a pharmaceutically acceptable salt thereof, or a solvate thereof as described above, in the manufacture of a medicament for preventing or treating a disease related to bacterial infection.

In some embodiments, the bacteria are Gram-negative bacteria.

In some embodiments, the bacteria are resistant to any polymyxin or antibiotic.

In some embodiments, the bacteria carry the mcr-1 gene.

In some embodiments, the bacteria are selected from the group consisting of *Escherichia coli, Acinetobacter baumannii, Pseudomonas aeruginosa,* and *Klebsiella pneumoniae.*

In some embodiments, the disease related to bacterial infection is selected from the group consisting of respiratory tract infection, pneumonia, bronchitis, wound infection, sepsis, and septicemia.

In another aspect, the present disclosure provides a method for treating a disease related to bacterial infection in a subject, comprising administering a therapeutically effective amount of the compound, a derivative thereof, a pharmaceutically acceptable salt thereof, or a solvate thereof as described above, or the pharmaceutical composition to the subject.

In some embodiments, the bacteria are Gram-negative bacteria.

In some embodiments, the bacteria are resistant to any polymyxin or antibiotic.

In some embodiments, the bacteria carry the mcr-1 gene.

In some embodiments, the bacteria are selected from the group consisting of *Escherichia coli, Acinetobacter baumannii, Pseudomonas aeruginosa,* and *Klebsiella pneumoniae.*

In some embodiments, the disease related to bacterial infection is selected from the group consisting of respiratory tract infection, pneumonia, bronchitis, wound infection, sepsis, and septicemia.

### BRIEF DESCRIPTION OF THE DRAWINGS

FIG. 1 is a schematic diagram showing the structure of the antimicrobial peptide provided herein.
FIG. 2 shows the binding of polymyxin E (Colistin) to modified and unmodified lipid A using PyMOL software.
FIG. 3 shows the binding of the antimicrobial peptide provided herein to modified and unmodified lipid A using PyMOL software.
FIG. 4 shows the minimum inhibitory concentration test results of the antimicrobial peptide provided herein against common Gram-negative bacteria.
FIG. 5 shows the cytotoxicity test results of the antimicrobial peptide provided herein and polymyxin E (Colistin).
FIG. 6 shows the nephrotoxicity test results of the antimicrobial peptide provided herein and polymyxin E (Colistin).
FIG. 7 shows the efficacy results of the antimicrobial peptide provided herein and polymyxin E (Colistin) in mice infected with polymyxin-resistant *Acinetobacter baumannii.*
FIG. 8 shows the mass spectrometry detection results (m/z) of the antimicrobial peptide provided herein.
FIG. 9 shows the HPLC purity test results of the antimicrobial peptide provided herein.

### DETAILED DESCRIPTION

Unless otherwise stated, all technical and scientific terms used herein have the same meanings as commonly understood by one of ordinary skill in the art. The term "or" refers to a single element among the listed alternative elements, unless the context clearly indicates otherwise. The term "and/or" refers to any one, any two, any three, any more, or all of the listed alternative elements.

The term "about" generally refers to a variation above or below the stated value by 10%, for example, by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

The term "comprise" or "include" means including the stated elements, integers, or steps, but does not preclude the presence of any other elements, integers, or steps. When the terms "comprise" or "include" are used herein, unless otherwise indicated, they also encompass instances consisting of the stated elements, integers, or steps. For example, an object stated to "comprise" a specific element is also intended to encompass an object consisting of that specific element.

"Derivative" of a compound as used herein refers to the compound linked to a substituent group via a covalent bond. For the compound of the following formula (I), the substituent group may substitute hydrogen atoms therein, for example, hydrogen atoms on the methyl, methylene, amino, imino, or hydroxyl groups.

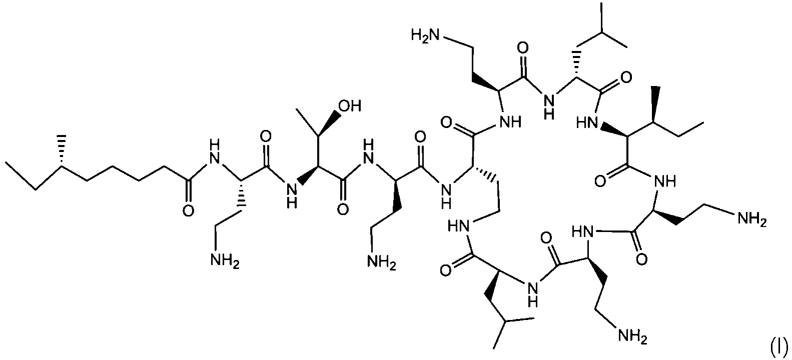

The substituent group can be, for example, a hydrocarbyl group, cyclic hydrocarbyl group, heterohydrocarbyl group, halogen, aryl group, heteroaryl group, etc. A hydrocarbyl group can be a linear or branched saturated or unsaturated hydrocarbyl group, preferably containing 1-12 carbon atoms. Examples of hydrocarbyl groups include methyl, ethyl, propyl, isopropyl, n-butyl, vinyl, etc. A hydrocarbyl group may be optionally substituted by one, two, three, or four substituents, which can be halogen, hydroxy, cyano, C1-12 alkyl, C3-7 cycloalkyl, aryl, heteroaryl, etc. In some embodiments, the hydrocarbyl group is unsubstituted. A cyclic hydrocarbyl group can be a cyclic saturated or unsaturated hydrocarbyl group having 3 to 18 (in some embodiments, 3 to 6) carbon atoms. In some embodiments, cyclic hydrocarbyl groups include, but are not limited to, cyclopropyl, cyclopentyl, cyclohexyl, cyclododecyl, etc. A cyclic hydrocarbyl group may be optionally substituted by one, two, three, or four substituents, which can be halogen, hydroxy, cyano, C1-12 alkyl, C3-7 cycloalkyl, aryl, heteroaryl, etc. In some embodiments, the cyclic hydrocarbyl group is unsubstituted. A heterohydrocarbyl group refers to a saturated or unsaturated hydrocarbyl group including heteroatoms, particularly halogen, N, O, or S. These heteroatoms can be incorporated into any part of the saturated or unsaturated hydrocarbyl group. Heteroatoms can be incorporated, for example, as hydroxy, C1-4 alkoxy, amino, mercapto (-SH) or similar groups. Examples of heteroalkyl groups include, but are not limited to: 2-methoxyethyl (-CH2CH2OCH3), 2-hydroxyethyl (-CH2CH2OH), hydroxymethyl (-CH2OH), 2-aminoethyl (-CH2CH2NH2), 2-dimethylaminoethyl (-CH2CH2N(CH3)2), benzyloxymethyl, thien-2-ylthiomethyl, etc. Halogen refers to fluorine (F), chlorine (CI), bromine (Br), or iodine (I). "Aryl" refers to an aromatic group, particularly phenyl or naphthyl. An aryl group may be optionally substituted by one, two, three, or four substituents, which can be halogen, hydroxy, cyano, C1-12 alkyl, C3-7 cycloalkyl, aryl, heteroaryl, etc. "Heteroaryl" refers to an aromatic group containing heteroatoms (particularly N, O, or S) in the aromatic ring. A heteroaryl group may be optionally substituted by one, two, three, or four substituents, which can be halogen, hydroxy, cyano, C1-12 alkyl, C3-7 cycloalkyl, aryl, heteroaryl, etc. Examples of heteroaryl groups include, but are not limited to: pyridine, thiophene, furan, pyrazole, pyrimidine, 2-pyridyl, 3-pyridyl, 4-pyridyl, 2-pyrimidinyl, 4-pyrimidinyl, 5-pyrimidinyl, 3-pyridazinyl, 4-pyridazinyl, 3-pyrazinyl, 4-oxo-2-imidazolyl, 2-imidazolyl, 4-imidazolyl, 3-isoxazolyl, 4-isoxazolyl, 5-isoxazolyl, 3-pyrazolyl, 4-pyrazolyl, 5-pyrazolyl, and the like. The term "optionally" as used herein means that the stated event or circumstance may, but need not, occur, and that the description includes instances where the event or circumstance occurs and instances where it does not. For example, "aryl optionally mono- or di-substituted by alkyl" means that the alkyl may be present but is not required, and includes both the case where the aryl is mono- or di-substituted by alkyl and the case where the aryl is not substituted by alkyl. In some cases, the substituent group can be a macromolecular polymer, such as polyethylene glycol (PEG) or polysaccharide (e.g., hyaluronic acid, chitosan, etc.) molecules.

In some cases, a derivative of the compound can be a prodrug of the compound, i.e., any substance that, when administered to a subject (such as a mammal, especially a human), is capable of releasing (e.g., degrading) in vivo to provide the compound. For example, a hydroxyl, mercapto, amido, or amino group in the compound can be linked to any group that can be cleaved in vivo to regenerate the corresponding free hydroxyl, amido, amino, or mercapto group. Examples of prodrugs include, but are not limited to, esters (e.g., acetate, formate, benzoate, phosphate, or phosphonate derivatives) of hydroxyl functional groups in the compounds provided herein, carbamates (e.g., N,N-dimethylaminocarbonyl), and the like.

"Pharmaceutically acceptable salt" as used herein refers to a salt form of the compound that retains the pharmacological activity of the compound. Such salts include acid addition salts formed with inorganic acids or organic acids, said inorganic acids being, for example, hydrochloric acid, hydrobromic acid, sulfuric acid, nitric acid, phosphoric acid, or similar acids; said organic acids being, for example, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 3-phenylpropionic acid, trimethylacetic acid, tert-butylacetic acid, lauryl sulfate, gluconic acid, glutamic acid, hydroxynaphthoic acid, salicylic acid, stearic acid, muconic acid, and the like.

"Solvate" as used herein refers to a complex formed between a solute (such as a compound of the present invention) and a solvent with a specific stoichiometry. The solvent can be those commonly used in the pharmaceutical field, for example, water, ethanol, acetic acid, and other solvents. The term "hydrate" refers to a solvate where the solvent molecule is water. The term "ethanolate" indicates that the solvent is ethanol.

"Bacterial infection" is used herein to mean that a subject may contain, harbor, or carry said bacteria, i.e., the bacteria may merely be present in or on the subject, which can include any part or location in or on the subject's body. Infection of the subject does not necessarily manifest as clinical disease (i.e., the infection causes clinical symptoms in the subject), although this can certainly be included. The subject here can be a subject suspected of having a bacterial infection or at risk of bacterial infection, i.e., it may be a subject merely exposed to bacteria, a subject showing clinical symptoms or signs of infection (if infection is suspected), or a subject susceptible to infection. In particular, the bacterial infection can be of a type known in routine clinical practice to be treated with polymyxin antibiotics. In some embodiments, the bacteria are identified as or suspected to be bacteria that respond to (i.e., are sensitive to) polymyxin antibiotics. In other embodiments, it may be determined that the infection (or more particularly, the bacteria in the infection) are resistant to one or more known polymyxin antibiotics.

The bacterial infection can be a nosocomial infection, a patient respiratory tract infection, for example, in patients with cystic fibrosis, chronic obstructive pulmonary disease, congestive obstructive airway disease/congestive obstructive airway pneumonia (COAD/COAP), pneumonia, emphysema, bronchitis, or sinusitis; a wound infection, particularly a chronic wound (including burns); a device-related infection associated with an implantable or prosthetic medical device, such as prosthetic valve endocarditis or line or catheter infection or artificial joint or tissue replacement or endotracheal or tracheostomy tube. Therefore, the site of infection can be a body cavity surface (e.g., teeth, gums, gingival crevices, periodontal pockets), reproductive tract (e.g., cervix, uterus, fallopian tubes), peritoneum, middle ear, prostate, urinary tract, vascular endothelium, eye, i.e., ocular tissue (such as conjunctiva, corneal tissue, tear ducts, lacrimal glands, eyelids), respiratory tract, lung tissue (e.g., bronchi and alveoli), heart valves, gastrointestinal tract, skin, scalp, nails, and inside wounds, particularly chronic wounds and surgical wounds, which may be local or internal wounds. Other surfaces include the exterior of organs, particularly those undergoing transplantation, such as heart, lung, kidney, liver, heart valves, pancreas, intestine, corneal tissue, arterial and venous grafts, and skin. Infection may also be present in body fluids (e.g., blood, plasma, serum, cerebrospinal fluid, gastrointestinal contents, semen, sputum, and other pulmonary secretions) and tissues (e.g., adrenal, liver, kidney, pancreas, pituitary, thyroid, immune, ovary, testis, prostate, endometrium, eye, breast, adipose, epithelial, endothelial, nerve, muscle, lung, epidermal, bone). Infection can also be found further on any "indwelling" medical or surgical equipment or device. This may include any type of line, including catheters (e.g., central venous and urinary catheters), prosthetic devices, such as heart valves, artificial joints, dentures, dental crowns, dental caps, and soft tissue implants (e.g., breast, hip, and lip implants). This includes any kind of implantable medical device (e.g., stents, intrauterine devices, pacemakers, cannulas (e.g., endotracheal or tracheostomy tubes), prostheses or prosthetic devices, lines, or catheters). An "indwelling" medical device may include a device any part of which is contained within the body, i.e., the device may be fully or partially indwelling. The infection can be acute, or it may be chronic, e.g., the infection persists for at least 5 days or at least 10 days, particularly at least 20 days, more particularly at least 30 days, most particularly at least 40 days. Examples of diseases related to bacterial infection include, but are not limited to, respiratory tract infection or conditions associated therewith, such as cystic fibrosis, pneumonia, chronic obstructive pulmonary disease (COPD), bronchitis, sinusitis, chronic wound infection (including burns), device-related infection associated with implantable or prosthetic medical devices, bacteremia, sepsis, septic shock, or septicemia. Examples of bacterial types that typically cause such infections include *Pseudomonas aeruginosa, Acinetobacter baumannii, Stenotrophomonas maltophilia, Burkholderia* (e.g., *Burkholderia cepacia*), *Escherichia coli, Klebsiella pneumoniae, Staphylococcus aureus,* Methicillin-resistant *Staphylococcus aureus* (MRSA), *Clostridium difficile, Mycobacterium tuberculosis, Enterococcus* and Vancomycin-resistant *Enterococcus,* and *Providencia stuartii.* Other important infections according to the present invention include those from *Bartonella* (e.g., *Bartonella henselae*), *Mycobacterium* (e.g., *Mycobacterium avium* complex (MAC), *Mycobacterium kansasii, Mycobacterium marinum, Mycobacterium ulcerans, Mycobacterium xenopi), Haemophilus influenzae* type B infection or *Legionella* (*e.g., Legionella pneumophila;* Legionnaires' disease), Leprosy (*Mycobacterium leprae*), Human granulocytic anaplasmosis (*Anaplasma phagocytophilum*), Brucellosis (*Brucella*), Meningococcal disease (*Neisseria meningitidis*), and Anthrax (*Bacillus anthracis*). The bacteria may be resistant to antibiotics, for example, the bacterial antibiotic resistance is to at least 1, 2, 3, 4, 5, 6, 7, 8, 9, or 10 antibiotics. Said antibiotics are, for example, aminoglycosides (e.g., amikacin, gentamicin, kanamycin, capreomycin, neomycin, netilmicin, streptomycin, tobramycin); β-lactams (e.g., carbacephems (e.g., loracarbef); first-generation cephalosporins (e.g., cefadroxil, cefazolin, cephalexin); second-generation cephalosporins (e.g., cefaclor, cefamandole, cefoxitin, cefprozil, cefuroxime); third-generation cephalosporins (e.g., cefixime, cefdinir, cefditoren, cefoperazone, cefotaxime, cefpodoxime, ceftazidime, ceftibuten, ceftizoxime, ceftriaxone); fourth-generation cephalosporins (e.g., cefepime); monobactams (e.g., aztreonam); macrolides (e.g., azithromycin, clarithromycin, dirithromycin, erythromycin, troleandomycin); monocyclics (e.g., aztreonam); penicillins (e.g., amoxicillin, ampicillin, carbenicillin, cloxacillin, dicloxacillin, nafcillin, oxacillin, penicillin G, penicillin V, piperacillin, ticarcillin); polypeptide antibiotics (e.g., bacitracin, polymyxin); quinolones (e.g., ciprofloxacin, enoxacin, gatifloxacin, levofloxacin, lomefloxacin, moxifloxacin, norfloxacin, ofloxacin, trovafloxacin); sulfonamides (e.g., sulfonamides, sulfacetamide, sulfamethizole, sulfasalazine, sulfisoxazole, trimethoprim-sulfamethoxazole); tetracyclines (e.g., demeclocycline, doxycycline, minocycline, oxytetracycline, tetracycline); glycylcyclines (e.g., tigecycline); carbapenems (e.g., imipenem, meropenem, ertapenem, doripenem, panipenem/betamipron, biapenem, PZ-601); and other antibiotics, including chloramphenicol; clindamycin; ethambutol; fosfomycin; isoniazid; linezolid; metronidazole; nitrofurantoin; pyrazinamide; quinupristin/dalfopristin; spectinomycin; fosfomycin; and vancomycin. Susceptibility to an antibiotic (as opposed to resistance and tolerance) can be measured in any convenient manner, e.g., using dilution susceptibility tests and/or disk diffusion tests. Preferably, the susceptibility of a bacterial strain to an antibiotic is expressed as the minimum inhibitory concentration (MIC) of that antibiotic for the microorganism (see Jorgensen et al., Manual of Clinical Microbiology, 7th ed., Washington, D.C: American Society for Microbiology, 1999; 1526-43). A person skilled in the art can use this information to determine whether the bacterial infection treated by the present invention is resistant to said antibiotic under these definitions. In some cases, the resistance is conferred by a plasmid carried by the bacteria, for example, said plasmid carries the mcr-1 gene. In other cases, the resistance is conferred by the mcr-1 gene carried in the bacterial genome.

The bacterial infection may include bacteria from any bacterial genus or species. For example, the bacteria may be Gram-positive or Gram-negative bacteria, or indeed Gram-indeterminate bacteria, wherein Gram-negative bacteria are important. Among Gram-negative bacteria, notable are Enterobacteriaceae bacteria and Gram-negative non-fermenting bacteria. Enterobacteriaceae include, but are not limited to, genera such as *Alishewanella, Alterococcus, Aquamonas, Aranicola, Azotivirga, Brenneria, Budvicia, B uttiauxella, Cedecea, Citrobacter, Cronobacter, Dickeya, Edwardsiella, Enterobacter, Erwi nia, Escherichia, Ewingella, Grimontella, Hafnia, Klebsiella, Leclercia, Leminorella, Moeller ella, Morganella, Obesumbacterium, Pantoea, Pectobacterium, Phlomobacter, Photorha bdus, Plesiomonas, Pragia, Proteus, Providencia, Rahnella, Raoultella, Salmonella, Samso nia, Serratia, Shigella, Sodalis, Tatumella, Trabusiella, Wigglesworthia, Xenorhabdus, Yers inia,* and *Yokenella.* Preferred enterobacterial genera include *Escherichia, Klebsiella, Salmonella, Shigella, Yersinia,* and *Providencia.* Non-fermenting Gram-negative bacteria include, but are not limited to, genera such as *Pseudomonas, Acinetobacter, Stenotrophomonas,*
*Burkholderia, Achromobacter, Alcaligenes, Bordetella, Brevundimonas, Comamonas, Eliz abethkingia, Methylobacterium, Moraxella, Ochrobactrum, Oligella, Psychrobacter, Ralst onia, Roseomonas, Shewanella, Sphingobacterium,* for example, *Pseudomonas aeruginosa, Acinetobacter baumannii, Stenotrophomonas maltophilia,* and *Burkholderiaspp.* Preferably, the bacteria can be selected from the genera *Pseudomonas, Acinetobacter, Stenotrophomonas, Burkholderia, Escherichia, Kle bsiella, Providencia, Streptococcus, Staphylococcus,* for example, *Pseudomonas aeruginosa, Acinetobacter baumannii, Stenotrophomonas maltophilia, Burkholderia* spp., *Escherichia coli, Klebsiella pneumoniae, Burkholderia cepacia, Burkholderia mallei, Burkholderia pseudomallei, Acinetobacter lwoffii*, *Providencia stuartii, Providencia rettgeri, Providencia alcalifaciens, Klebsiella oxytoca, Pseudomonas anguilliseptica, Pseudomonas oryzihabitans, Pseudomonas plecoglossicida, Pseudomonas luteola,* and Methicillin-resistant *Staphylococcus aureus*(MRSA).

When referring to a pharmaceutical composition, the "pharmaceutically acceptable carrier" used refers to a solid or liquid diluent, filler, antioxidant, stabilizer, or other substance that can be safely administered to an animal or human body, which is suitable for administration to humans and/or animals without excessive adverse side effects, while being suitable for maintaining the viability of the drug or active agent contained therein. Depending on the route of administration, various different carriers well known in the art can be used, including, but not limited to, saccharides, starches, cellulose and its derivatives, maltose, gelatin, talc, calcium sulfate, vegetable oils (e.g., castor oil), synthetic oils, polyalcohols, alginic acid, phosphate buffers, emulsifiers, isotonic saline, and/or pyrogen-free water, etc. Routes of administration that can be used include, for example, oral, intravenous infusion, intramuscular injection, subcutaneous injection, intraperitoneal, rectal, sublingual, or via inhalation, transdermal, etc. Accordingly, the pharmaceutical composition comprising the compound, a derivative thereof, a pharmaceutically acceptable salt thereof, or a solvate thereof provided herein can be formulated into any suitable dosage form, such as tablets, granules, powders, capsules, injection preparations, suppositories, eye drops, topical ointments, creams, medicated oils, or sprays, etc.

The compound of formula (I) provided herein, a derivative thereof, a pharmaceutically acceptable salt thereof, or a solvate thereof can be used in combination with an additional therapeutically active agent. This additional active agent can be in the same pharmaceutical formulation (e.g., pharmaceutical composition) as said compound, a derivative thereof, a pharmaceutically acceptable salt thereof, or a solvate thereof, or in a different pharmaceutical formulation. They can be administered together, or separately (i.e., separately, sequentially, or simultaneously). Therefore, another aspect of the present invention provides a product (e.g., a pharmaceutical combination or kit) comprising the compound of formula (I) as defined herein, a derivative thereof, a pharmaceutically acceptable salt thereof, or a solvate thereof and an additional therapeutically active agent as a combined preparation for separate, sequential, or simultaneous use in treating or preventing a bacterial infection in a subject. In some embodiments, the additional active agent is, for example, an antimicrobial agent (e.g., antibacterial agent, antifungal agent, antiviral agent, antibiotic as mentioned above), an immunostimulant, a corticosteroid, a non-steroidal anti-inflammatory drug (NSAID), a bronchodilator, etc.

"Subject" includes an animal, such as a mammal, including (but not limited to) primates, rodents, simians, felines, canines, equines, bovines, porcines, sheep, goats, mammalian laboratory animals, mammalian farm animals, mammalian sport animals, and mammalian pets. The subject can be male or female and can be a subject of any suitable age, including infant, juvenile, adolescent, adult, and elderly subjects. In some instances, a subject refers to an individual requiring diagnosis or treatment of a disease or condition. In some instances, a subject receiving diagnosis or treatment can be a patient who has a condition associated with said diagnosis or treatment, or is at risk of developing the condition. In specific instances, the subject is a human, such as a human patient. This term is generally interchangeable with "patient", "test subject", "treatment subject", etc.

When referring to the treatment of a disease, a "therapeutically effective amount" refers to an amount of an active molecule (such as the compound provided herein) sufficient to elicit the biological or medical response desired by a clinician in a subject. The "effective amount" upon administration can be determined by a person skilled in the art based on factors such as the route of administration, the subject's body weight, age, condition, etc. For example, for a general drug, a typical daily dose range can be from 1 µg to 100 mg of active ingredient per kg of body weight. Administration methods for the active molecule provided herein (such as the compound or a derivative thereof provided herein) include, but are not limited to, injection, e.g., intravenous, intramuscular, intra-arterial, subcutaneous, intraperitoneal, etc.

Polymyxin is a group of polypeptide antibiotics produced by *Paenibacillus polymyxa,* which inhibit most Gram-negative bacteria. The peptide chain of polymyxin typically consists of 10 amino acids, structurally forming a linear tripeptide linked to a cyclic heptapeptide. Differences in the type and configuration of amino acids at different positions form different polymyxin homologs. Polymyxin amino acid sequence: L-Dab-L-Thr-3-L-Dab-L-Dab-6-7-L-Dab-L-Dab-L-Thr, wherein the differences at the 3rd, 6th, and 7th amino acid positions determine the type of homolog (Table 1). Currently, only polymyxin B and polymyxin E are used clinically. They have almost identical primary sequences, differing only at the 6th amino acid. Polymyxin B has D-Phe at the 6th amino acid position, while polymyxin E has D-Leu.

**Table 1 Sequences of amino acids at positions 3, 6, 7 and the fatty acyl tail of polymyxin homologs**

| **Polymyxin** | **Position 3** | **Position 6** | **Position 7** | **Fatty Acyl Tail** |
|---|---|---|---|---|
| polymyxins A | | | | |
| P-A1 | D-Dab | D-Leu | L-Thr | 6-MOA |
| P-A2 | D-Dab | D-Leu | L-Thr | 6-MHA |

| polymyxins B | | | | |
|---|---|---|---|---|
| P-B1 | L-Dab | D-Phe | L-Leu | 6-MOA |
| P-B2 | L-Dab | D-Phe | L-Leu | 6-MHA |
| P-B3 | L-Dab | D-Phe | L-Leu | OA |
| P-B4 | L-Dab | D-Phe | L-Leu | HA |
| P-B5 | L-Dab | D-Phe | L-Leu | NA |
| P-B6 | L-Dab | D-Phe | L-Leu | 3-OH-6-MOA |
| lie-P-B1 | L-Dab | D-Phe | L-Ile | 6-MOA |

| polymyxins C | | | | |
|---|---|---|---|---|
| P-C | L-Dab | D-Phe | L-Thr | 6-MOA |
| polymyxins D | | | | |
| P-D1 | D-Ser | D-Leu | L-Thr | 6-MOA |
| P-D2 | D-Ser | D-Leu | L-Thr | 6-MHA |

| polymyxins E | | | | |
|---|---|---|---|---|
| P-E1 (Colistin) | L-Dab | D-Leu | D-Leu | 6-MOA |
| P-E2 | L-Dab | D-Leu | D-Leu | 6-MHA |
| P-E3 | L-Dab | D-Leu | D-Leu | OA |
| P-E4 | L-Dab | D-Leu | D-Leu | HA |
| P-E7 | L-Dab | D-Leu | D-Leu | 7-MOA |

The structure of the polymyxin-like compound provided herein (hereinafter referred to as the novel antimicrobial peptide or antimicrobial peptide) is as follows:

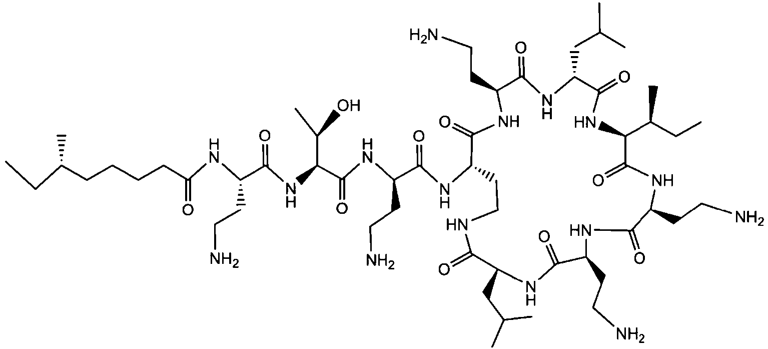

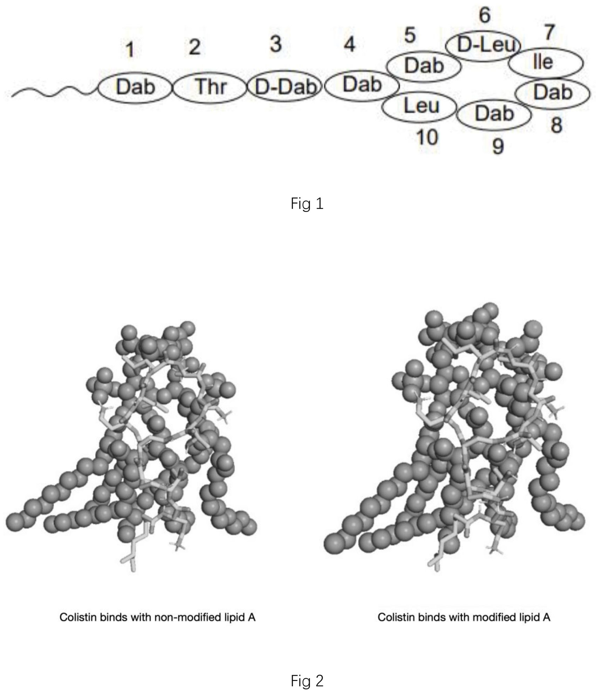

The primary sequence of this antimicrobial peptide can be represented as: {6-ME-Octanoic acid}-{Dab}-{Thr}-{D-Dab}-{Dab}-{Dab}-{D-Leu}-{Ile}-{Dab}-{Dab}-{Leu} (see Figure 1). Its amino terminus is S-6-methyloctanoic acid, the first 3 amino acids are linearly connected, and the last 7 amino acids form a ring.

Using clinical Polymyxin E (Colistin) as an example, the structure of the novel antimicrobial peptide provided herein is compared with existing polymyxins. As shown in the structural formula below, the novel antimicrobial peptide provided herein differs from Polymyxin E (Colistin) in the types of amino acids at positions 7 and 10, and in the conformation of the amino acid at position 3. The amino acids at positions 3, 7, and 10 of the novel antimicrobial peptide provided herein are D-Dab, L-Ile, and L-Leu, respectively, whereas those of Polymyxin E (Colistin) are L-Dab, D-Leu, and L-Thr.

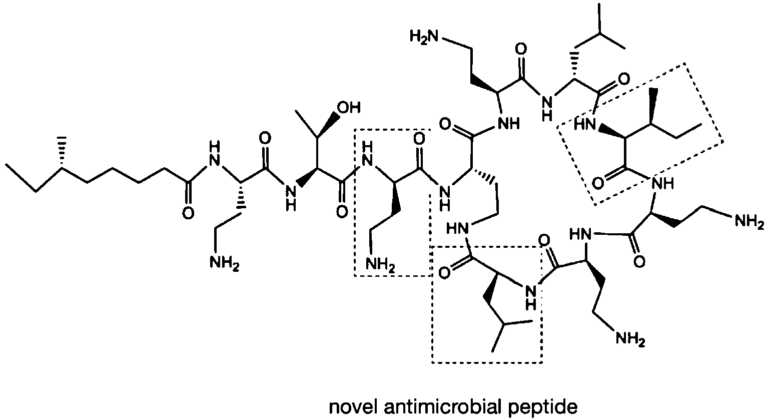

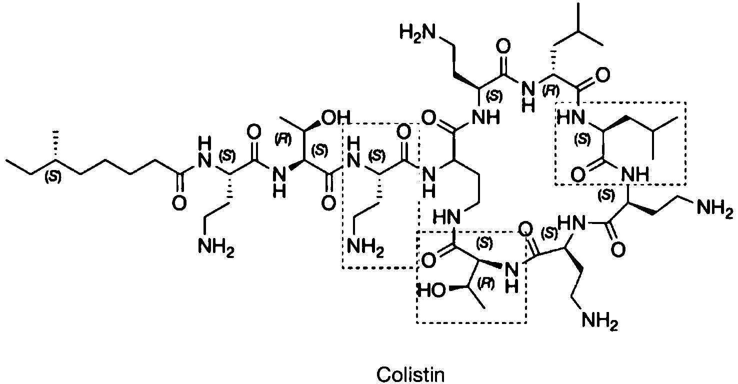

The novel antimicrobial peptide provided herein possesses a significant ability to inhibit polymyxin-resistant bacteria. We used PyMOL to simulate the binding of Polymyxin E (Colistin) or the novel antimicrobial peptide to lipid A, thereby analyzing the mechanism of action of the novel antimicrobial peptide. As shown in Figure 2 (left panel), Polymyxin E (Colistin) binds to the two negatively charged phosphate groups of lipid A. When lipid A is modified with phosphoethanolamine (Figure 2, right panel), the negative charge of lipid A is reduced, and Polymyxin E (Colistin) has only one binding site on lipid A, significantly reducing its binding affinity.

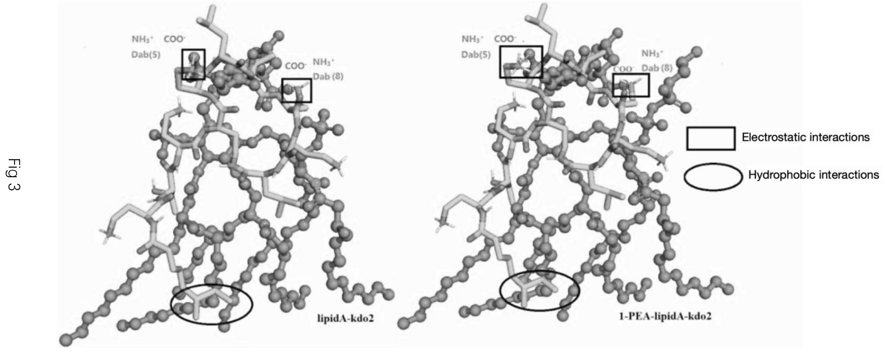

Figure 3 shows potential interaction models between the antimicrobial peptide and the Gram-negative bacterial membrane component Kdo2-Lipid A, and between the antimicrobial peptide and the membrane component 1-PEA-Kdo2-Lipid A of Gram-negative bacteria carrying mcr-1. As seen in Figure 3, the positively charged Dab (5) of the antimicrobial peptide electrostatically interacts with the carboxyl group of Kdo2 in the Gram-negative bacterial membrane component Kdo2-Lipid A, and the positively charged Dab (8) electrostatically interacts with the carboxyl group of Kdo1 in Kdo2-Lipid A. Simultaneously, the fatty acid molecule of the antimicrobial peptide engages in hydrophobic interactions with the fatty acid molecules of Kdo2-Lipid A. These two types of interactions likely confer Gram-negative antibacterial activity to the antimicrobial peptide.

Although the phosphoethanolamine transferase encoded by mcr-1 adds a modification to the phosphate group at position 1 of the Gram-negative bacterial membrane component Kdo2-Lipid A, neutralizing the negative charge of the phosphate group, it does not affect the binding of Dab (5) and Dab (8) to Kdo2 and Kdo1, respectively. Meanwhile, the fatty acid molecule of the antimicrobial peptide engages in hydrophobic interactions with the fatty acid molecules of 1-PEA-Kdo2-Lipid A. These two effects likely confer antibacterial activity to the antimicrobial peptide against Gram-negative bacteria carrying mcr-1.

Due to the strong binding capacity of the novel antimicrobial peptide to the negative charges on lipid A, the antimicrobial peptide shares a similar antibacterial mechanism with polymyxins and can inhibit the growth of common Gram-negative bacteria, including *Escherichia coli, Acinetobacter baumannii, Pseudomonas aeruginosa,* and *Klebsiella pneumoniae.* Furthermore, the novel antimicrobial peptide still maintains a very high binding capacity to modified lipid A, enabling it to have a strong inhibitory effect on polymyxin-resistant bacteria, such as *Acinetobacter baumannii, Pseudomonas aeruginosa,* and *Klebsiella pneumoniae* containing the mcr-1 plasmid.

### Example 1: Detection of the Inhibitory Effect of the Antimicrobial Peptide Provided Herein on Common Gram-Negative Bacteria

### 1.1 Experimental Procedure

The pathogenic bacteria used for testing were all sourced from the intensive care unit of Gulou Hospital, isolated and cultured from clinical samples of patients with severe pneumonia. The strain species were identified by the Microbiology Laboratory of Gulou Hospital. The Gram-negative bacteria here include *Escherichia coli, Pseudomonas aeruginosa, Acinetobacter baumannii, Klebsiella pneumoniae,* etc.

### 1.1.1 Preparation of Test Strains

A single colony of the test bacteria was inoculated into 5 mL of medium and cultured overnight to saturation. 10 µL of bacterial culture was aspirated and added to 990 µL of fresh medium, mixed well, to obtain a 100-fold diluted bacterial suspension. Then, 20 µL of the 100-fold diluted bacterial suspension was aspirated and added to 980 µL of fresh medium, mixed well. The bacteria obtained at this point were diluted 5000-fold.

### 1.1.2 Dilution of Antimicrobial Peptide

The antimicrobial peptide was dissolved in Dimethyl sulfoxide (DMSO) to obtain a stock solution with a concentration of 12.8 mg/mL. Using DMSO, a two-fold serial dilution was performed for 10 consecutive times.

### 1.1.3 Antibacterial Activity Test of Antimicrobial Peptide

Add the 11 gradients of antimicrobial peptide diluted in step 1.1.2 to a 96-well plate, respectively, with the last well containing the same volume of DMSO. Then, add 48 µL of fresh medium to each well. Subsequently, add the 5000-fold diluted bacteria from step 1.1.1 to each well. Mix well. Incubate at 37°C for 16 hours. Visually determine the concentration at which bacterial growth begins to be inhibited; this is the minimum inhibitory concentration (MIC).

### 1.2 Experimental Results

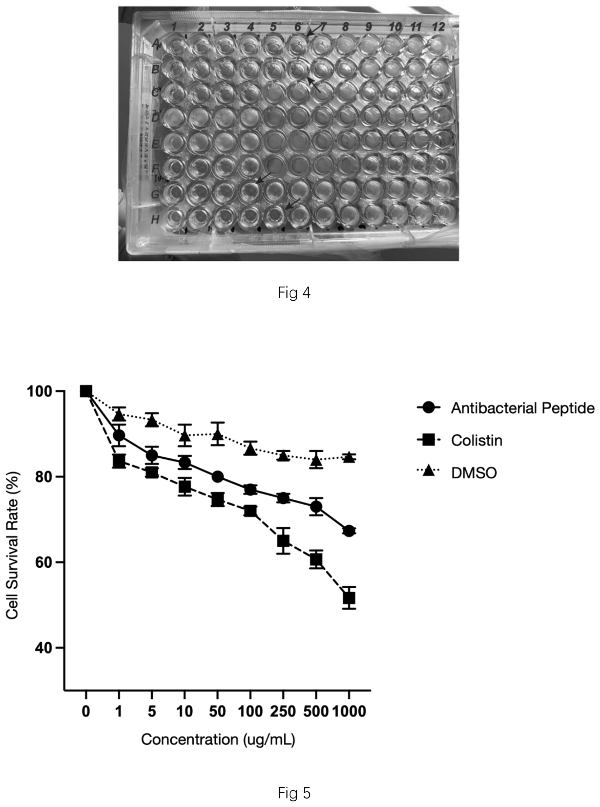

As shown in Figure 4, the antimicrobial peptides provided in this study were all capable of inhibiting the growth of common *E. coli, Pseudomonas aeruginosa, Acinetobacter baumannii,* and *Klebsiella pneumoniae.*
- A1-A12 were cultured with *E coli* using antimicrobial peptide concentrations of 128, 64, 32, 16, 8, 4, 2, 1, 0.5, 0.25, 0.135, and 0 µg/mL, respectively.
- B1-B12 were cultured with *Pseudomonas aeruginosa* using antimicrobial peptide concentrations of 128, 64, 32, 16, 8, 4, 2, 1, 0.5, 0.25, 0.135, and 0 µg/mL, respectively.
- G1-G12 were cultured with *Acinetobacter baumannii* using antimicrobial peptide concentrations of 128, 64, 32, 16, 8, 4, 2, 1, 0.5, 0.25, 0.135, and 0 µg/mL, respectively.
- H1-H12 were cultured with *Klebsiella pneumoniae* using antimicrobial peptide concentrations of 128, 64, 32, 16, 8, 4, 2, 1, 0.5, 0.25, 0.135, and 0 µg/mL, respectively.
- D1-D2: Culture of *E*. *coli* without antimicrobial agent.
- D3-D4: Culture of *Pseudomonas aeruginosa* without antimicrobial agent.
- E1-E2: Culture of *Acinetobacter baumannii* without antimicrobial agent.
- E3-E4: Culture of *Klebsiella pneumoniae* without antimicrobial agent.
- Other single wells did not contain any samples.
- The blue arrows indicate the wells corresponding to the Minimum Inhibitory Concentration (MIC).

The specific Minimum Inhibitory Concentrations (MICs) are as follows:
- 4 µg/mL (*E. coli*)
- 4 µg/mL (*Pseudomonas aeruginosa*)
- 16 µg/mL (*Acinetobacter baumannii*)
- 8 µg/mL (*Klebsiella pneumoniae*)

### Example 2: Inhibition of Polymyxin-Resistant Bacteria by the Novel Antimicrobial Peptide

### 2.1 Experimental Procedure

The polymyxin-resistant pathogenic bacteria used for testing were all sourced from the hospital intensive care unit, isolated and cultured from clinical samples of patients with severe pneumonia. The strain species were identified by the hospital microbiology laboratory, and the presence of the mcr-1 gene in the strains was confirmed by high-throughput sequencing.

The culture procedure fully referenced Example 1.

### 2.2 Experimental Results

**Table 2 Minimum Inhibitory Concentrations (µg/mL) of Polymyxin E (Colistin) and the Antimicrobial Peptide against Bacteria Containing the MCR-1 Plasmid**

| | ***A. baumannii 1*** | ***A. baumannii 2*** | ***P. aeruginosa 1*** | ***P. aeruginosa 2*** | ***E coli 1*** | ***E coli 2*** | ***K. pneumoniae 1*** | ***K. pneumoniae 2*** |
|---|---|---|---|---|---|---|---|---|
| **Colistin** | 128 | 128 | 64 | 128 | 128 | 256 | 64 | 128 |
| **Antimicrobial Peptide** | 4 | 2 | 2 | 1 | 2 | 2 | 1 | 2 |

Each bacterial species in the table was tested using 2 strains, denoted as "1" and "2" respectively.

As shown in Table 2, for *Escherichia coli, Pseudomonas aeruginosa, Acinetobacter baumannii,* and *Klebsiella pneumoniae* containing the MCR-1 gene, the minimum inhibitory concentrations of the antimicrobial peptide were significantly lower than those of Polymyxin E (Colistin), demonstrating the higher inhibitory activity of the antimicrobial peptide against polymyxin-resistant strains.

### Example 3: Cytotoxicity Test of the Antimicrobial Peptide on Human Cells

### 3.1 Experimental Procedure

### 3.1.1 Cell Culture and Drug Treatment

HEK293 cells were cultured in Dulbecco's Modified Eagle's Medium (containing 10% fetal bovine serum). After passaging at a density of 2500 cells per well in a 96-well plate, they were cultured in a 37°C incubator with 5% CO2 for 24 hours. Then, different concentrations (0~1000 µg/mL) of the antimicrobial peptide were added to each well. After 48 hours of culture, the medium was removed, and 3-(4,5-Dimethyl-2-thiazolyl)-2,5-diphenyl-2H-tetrazolium bromide (MTT) solution (0.45 mg/mL) was added to each well. After incubating for 3 hours, the solution was aspirated. 100 µL of solubilization solution (40% DMF, 16% SDS, and 2% acetic acid in water) was added to dissolve the precipitated formazan crystals.

### 3.1.2 Statistical Analysis of Cell Viability

The absorbance of each well was measured at OD570 nm using a microplate reader (Epoch Microplate Spectrophotometer, BioTek). Polymyxin E (Colistin) was used as a positive control, and DMSO as a negative control. IC50 values, i.e., the concentration of each compound required to produce 50% inhibition of cell growth compared to the no-compound control, were calculated using GraphPad Prism v.9.0.

### 3.2 Experimental Results

As shown in Figure 5, similar to Polymyxin E (Colistin), the concentration of the antimicrobial peptide required to produce 50% inhibition of cell growth was greater than 512 µg/mL, indicating very low cytotoxicity of the antimicrobial peptide.

### Example 4: Nephrotoxicity Test of the Antimicrobial Peptide in Mice

### 4.1 Experimental Procedure

### 4.1.1 Mouse Rearing and Drug Treatment

This experiment used 6-week-old CD1 female mice (20-25 grams). The room had a 12-hour light cycle, temperature of 21°C, and humidity of 30%. After 3 days of acclimatization training, the mice were randomly divided into three groups (n=6 per group): vehicle group, Polymyxin E (Colistin) group, and antimicrobial peptide group.

Mice were subcutaneously injected with 100 µL of 0.9% saline (vehicle group), 20 mg/kg Polymyxin E (Colistin), or 20 mg/kg antimicrobial peptide, respectively, for 7 consecutive days. Blood samples were collected 12 hours after the last injection.

### 4.1.2 Determination of Kidney Injury in Mice

NGAL (Neutrophil Gelatinase-Associated Lipocalin) is commonly used as an indicator of kidney injury. Here, we used a commercially available Mouse NGAL ELISA Kit (Beyotime, PN758) to measure serum NGAL concentration. The detection process is briefly described as follows:
a. Leave whole blood at room temperature for 30 min-2 h, then centrifuge at 1000 g for 10 minutes at 4°C. Aspirate the yellow supernatant to obtain serum.
b. Prepare standard diluent and standards: Dilute the standard diluent (5X) to 1X with deionized water. According to the instructions, add diluent to the standard vial, incubate at room temperature for 15 minutes, to achieve a final standard concentration of 5000 pg/mL.
c. Prepare wash buffer: Dilute the wash buffer (20X) to 1X with deionized water.
d. Perform serial dilution of the standard using the standard diluent, finally obtaining six standard concentrations: 5000, 2500, 1250, 625, 312.5, 156.5 pg/mL, and add them sequentially to the pre-coated plate wells. Simultaneously, add the standard diluent directly as the 0 pg/mL control. Note: Standards must be prepared and a standard curve plotted for each experiment; it is also recommended to set up a background correction well, i.e., a well containing only TMB solution and stop solution.
e. Add samples or different concentration standards (100 µL/well) to the corresponding wells, seal with a plate sealer (transparent), and incubate at room temperature for 120 min.
f. Wash the plate 5 times, and after the last wash, pat dry on thick absorbent paper.
g. Add biotinylated antibody (100 µL/well). Seal with a plate sealer (transparent) and incubate at room temperature for 60 min.
h. Wash the plate 5 times, and after the last wash, pat dry on thick absorbent paper.
i. Add Horseradish Peroxidase (HRP)-labeled Streptavidin (100 µL/well). Seal the reaction wells with a plate sealer (white) and incubate at room temperature protected from light for 20 min.
j. Wash the plate 5 times, and after the last wash, pat dry on thick absorbent paper.
k. Add the chromogenic substrate TMB solution (100 µL/well), seal with a plate sealer (white), and incubate at room temperature protected from light for 15-20 min.
l. Add stop solution (50 µL/well), mix well, and immediately measure the absorbance at 450 nm.
m. Calculate the standard curve and convert the NGAL concentration in the samples based on the standard curve.

### 4.2 Experimental Results

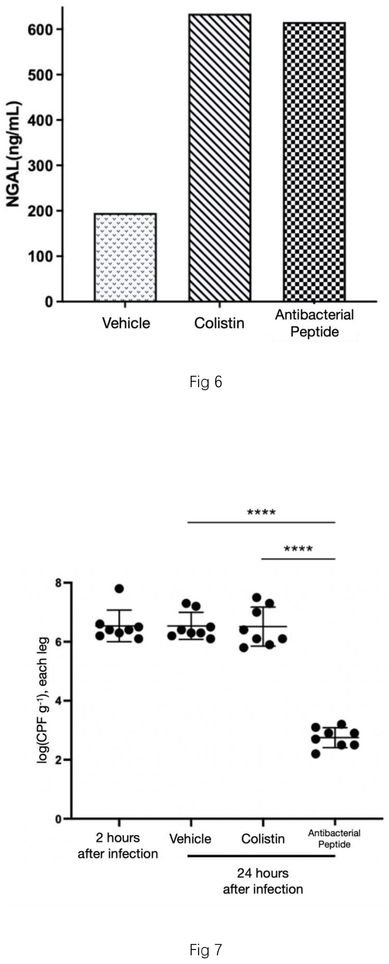

As shown in Figure 6, there was no significant difference in the NGAL levels induced by injection of the antimicrobial peptide compared to Polymyxin E (Colistin) in mice, meaning their degree of nephrotoxicity is highly similar. This indicates that the antimicrobial peptide has a safety profile similar to that of Polymyxin E (Colistin).

### Example 5: Efficacy of the Antimicrobial Peptide in Mice Infected with Polymyxin-Resistant Acinetobacter baumannii

### 5.1 Experimental Procedure

### 5.1.1 Construction of the Neutropenic Mouse Model

All mice used in this part passed the ethical review of animal research at Nanjing University. To avoid the influence of the mouse's innate immune system on the intrinsic activity of the antimicrobial peptide, we constructed a neutropenic mouse model. This experiment used 6-week-old CD1 female mice (20-25 g). Mice were randomly housed in individually ventilated cages and maintained according to the standards of the Association for Assessment and Accreditation of Laboratory Animal Care International. The room was set to a 12-hour light cycle, temperature of 21°C, and humidity of 30%. On day -4 and day -1 before bacterial infection, mice were intraperitoneally injected with 150 mg/kg and 100 mg/kg cyclophosphamide, respectively, to induce neutropenia.

### 5.1.2 Culture of Polymyxin-Resistant Acinetobacter baumannii and Infection

A single colony of polymyxin-resistant *Acinetobacter baumannii* was picked and cultured overnight at 37°C in cation-adjusted Mueller-Hinton (MH) broth containing 50 µg/mL gentamicin. The culture was centrifuged, the supernatant aspirated, and the bacteria gently washed twice with sterile saline. The OD value was checked at 600 nm, and dilutions were made. A 0.05 mL bacterial suspension was injected intramuscularly into both thighs of the mice, providing approximately 1.0 x 10^6 CFU of bacteria per thigh.

### 5.1.3 Determination of Antimicrobial Peptide Efficacy

At 2, 8, 14, and 24 hours post-infection, mice were subcutaneously injected with 100 µL of vehicle (0.9% saline), Polymyxin E (Colistin) (20 mg/kg), or the antimicrobial peptide (20 mg/kg). At 2 hours post-infection, untreated control infected mice (n=4 mice, n=8 thighs) were humanely euthanized by CO2 asphyxiation to determine the initial bacterial burden in the thighs. All mice were closely monitored post-infection for signs of illness. Any abnormal clinical symptoms were recorded. At the experimental endpoint, 24 hours post-infection, mice were euthanized by CO2 asphyxiation (4 mice/8 thighs per condition). Thigh muscles were processed aseptically, weighed, homogenized, and plated for CFU counts on MH agar containing 50 µg/mL gentamicin to quantify the bacterial burden. Efficacy was determined by the reduction in bacterial burden in the thighs compared to the treated control group. All graphical data were statistically analyzed using GraphPad Prism software (Prism v.9). Differences in burden between test and control groups were assessed using one-way analysis. A P-value < 0.05 was considered statistically significant.

### 5.2 Experimental Results

Neutropenic mice were infected with polymyxin-resistant *Acinetobacter baumannii* and then treated with Polymyxin E (Colistin) and the antimicrobial peptide, respectively. As shown in Figure 7, Polymyxin E (Colistin) did not reduce the count of *Acinetobacter baumannii,* whereas the antimicrobial peptide exhibited potent antibacterial activity, with a significant reduction in colony-forming units (CFU) compared to the vehicle control group and the Polymyxin E (Colistin) treatment group. These results indicate that the antimicrobial peptide has a significant inhibitory effect on polymyxin-resistant *Acinetobacter baumannii.*

### Example 6: Synthesis and Detection Process of the Antimicrobial Peptide

### 6.1 Antimicrobial Peptide Synthesis Process

1. **Swelling the Resin**
   Place Fmoc-Leu-CTC Resin into a column reactor. Add dichloromethane (10 ml/g), mix, and soak for 30 min to allow the resin to fully swell.
2. **Removal of the Resin Protecting Group**
   Vacuum dry the swelling solvent. Add 20% Piperidine/N,N-Dimethylformamide (DMF) (10 ml/g), mix using nitrogen bubbling for 30 min. After drying the deprotection reagent, wash with DMF (10 ml/g) 6 times, with nitrogen bubbling for 1 min and vacuum drying for 1 min each time. Take a small amount of resin for a deprotection test. Add 1 ml ninhydrin test reagent to the resin, place the test tube in a heater above 120°C for 3 min, remove and observe the resin color. If the resin color darkens, it indicates successful deprotection, and the next coupling reaction can proceed; if the resin is light red or yellow, extend the deprotection time.
3. **Coupling the Second Amino Acid**
   Dissolve 3 equivalents of Fmoc-Dab(Boc)-OH and 3 equivalents of the coupling reagent OXYMA in DMF. Then add 3 equivalents of the coupling reagent DIC, react for 5 min for activation. Add the above solution to the reactor, react with nitrogen bubbling for 1-2 h to complete the coupling reaction. Then check the resin color to detect if coupling was successful. No significant change indicates successful coupling, yielding Fmoc-Dab-Leu-CTC resin.
4. **Prepare the Resin for Coupling the Next Amino Acid**
   After drying the reaction reagents, wash the resin obtained in step 3) with DMF (10 ml/g) 4 times, with nitrogen bubbling for 1 min and vacuum drying for 1 min each time, to remove residual solvent. Consistent with steps 1) and 2), perform resin swelling, removal of the Fmoc protecting group, and deprotection effect testing.
5. **Coupling the Third Amino Acid**
   Consistent with step 3), dissolve 3 equivalents of Fmoc-Dab(Boc)-OH and 3 equivalents of OXYMA in DMF, and add 3 equivalents of DIC for activation for 5 min. Add this to the reactor, react with nitrogen bubbling for 1-2 h to complete the second coupling reaction. Observe the resin color change, confirming the obtainment of Fmoc-Dab-Dab-Leu-CTC resin.
6. **Cycling Steps**
   Repeat steps 1) to 5) to couple the amino acids in the sequence from right to left, respectively: Fmoc-Ile-OH, Fmoc-D-Leu-OH, Fmoc-Dab(Boc)-OH, Fmoc-Dab(ivdde)-OH, Fmoc-D-Dab(Boc)-OH, Fmoc-Thr(tBu)-OH, Fmoc-Dab(Boc)-OH, obtaining Fmoc-Dab-Thr-{D-Dab}-Dab-Dab-{D-Leu}-Ile-Dab-Dab-Leu-CTC resin.
7. **Coupling the Special Raw Material 6-ME-Octanoic Acid**
   For the resin obtained in step 6), repeat steps 1) and 2) to prepare for coupling 6-ME-Octanoic Acid. Dissolve 3 equivalents of 6-ME-Octanoic Acid and 3 equivalents of OXYMA in DMF, then add 3 equivalents of DIC for activation for 5 min, then add this to the reactor, react with nitrogen bubbling for 1-2 h to complete the coupling.
8. **Prepare the Resin for Cyclization**
   Vacuum dry the reaction reagents. Wash with DMF (10 ml/g) 4 times, with nitrogen bubbling for 1 min and vacuum drying for 1 min each time.
9. **Cyclization**
   The raw material for amino acid number 5 was Fmoc-Dab(ivdde)-OH. Here, add 5% Hydrazine Hydrate/DMF (12 ml/g), perform nitrogen bubbling 3 times, 10 min each time, to remove the lvdde protecting group from the side chain of amino acid number 5, without affecting the side chains of other amino acids. Wash and remove the protecting group. Dissolve 2 equivalents of the coupling reagent HATU in DMF, then add 4 equivalents of the coupling reagent DIEA to the reactor, react with nitrogen bubbling for 1-2 h to complete the cyclization. Observe the resin color; no significant change in resin color indicates successful cyclization.
10. **Washing and Drying the Antimicrobial Peptide**
   Vacuum dry the reagents. Wash with DMF (10 ml/g) 5 times, with nitrogen bubbling for 1 min and vacuum drying for 1 min each time, then wash the resin with methanol (12 ml/g) 5 times, vacuum dry for 1 h.
11. **Cleavage from Resin**
   Transfer the resin obtained in step 10) into a boat reactor, add 10 ml/g cleavage cocktail, and cleave at room temperature for 3 h. Then filter the reaction solution and add it to 10 times the volume of ice-cold diethyl ether. Centrifuge to precipitate, and wash with ice-cold diethyl ether 3 times, to obtain the crude white peptide solid.
12. **Purification of the Crude Antimicrobial Peptide**
   Perform initial purification of the antimicrobial peptide using a Trifluoroacetic acid (TFA)/water system. Purification conditions: use 0.065% TFA in water as mobile phase A, and 0.05% TFA in acetonitrile as mobile phase B. Perform separation at room temperature on a C18 column at a flow rate of 15 ml/min. Monitor the peaks at 220 nm, collect samples before the main peak, the main peak itself, and after the main peak separately for preliminary mass spectrometry testing. Purify the collected product again using an HCl/water system as the mobile phase. Purification conditions: use 0.065% HCl in water as mobile phase A, and 0.05% TFA in acetonitrile as mobile phase B. Perform separation at room temperature on a C18 column at a flow rate of 15 ml/min. Monitor the peaks at 220 nm, and collect the main peak as the target sample.
13. **Lyophilization and Packaging**
   Transfer the purified qualified fractions into a lyophilization tray, cover it, and place it in a freeze dryer for lyophilization. After lyophilization is complete, remove the tray, weigh and package the peptide sample, and store at -20°C.

### 6.2 Antimicrobial Peptide Detection Process

### 1. Antimicrobial Peptide Identification

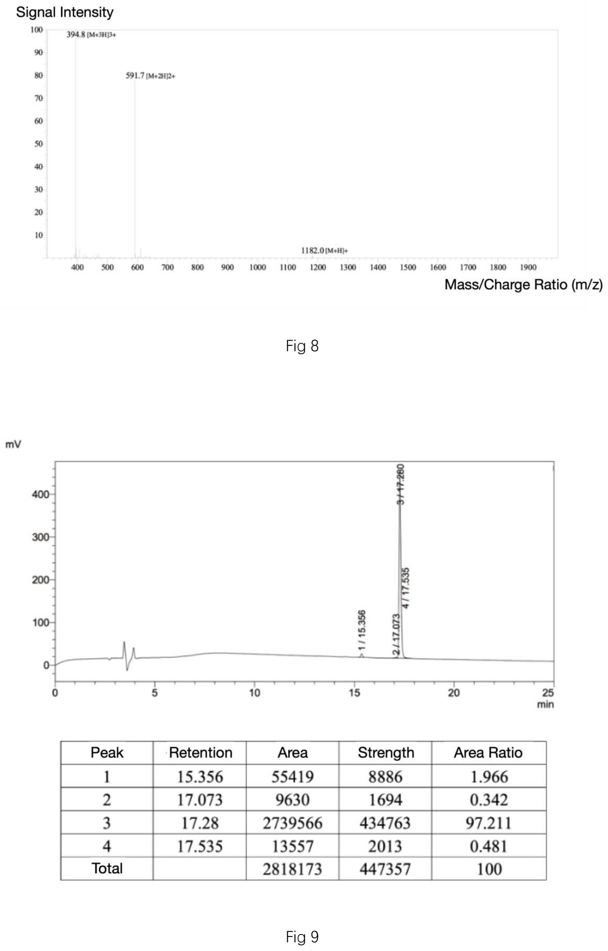

Dissolve the antimicrobial peptide in water, adjust the concentration to 1 mg/mL, then perform Mass Spectrum detection using a SHIMADZU LCMS-2020 instrument in positive ion mode. The mass spectrum results show the mass-to-charge ratio (m/z, m is molecular weight, z is charge number), so the product molecular weight equals the product of m/z and z. Since hydrogen atoms (H) equivalent to the charge number are added in positive ion mode, the molecular weight of the hydrogen atoms must be subtracted to obtain the final molecular weight of the product. The formula is: Relative Molecular Weight MW = (m/z) * z - z. As shown in Figure 8, distinct m/z peaks were detected under 3 different charge conditions.

When z=1, product MW = 1182.0 * 1 - 1 = 1181.0.

When z=2, product MW = 591.7 * 2 - 2 = 1181.4.

When z=3, product MW = 394.8 * 3 - 3 = 1181.4.

Based on calculation from the sequence and structure of the antimicrobial peptide, the molecular weight is 1181.4, consistent with the obtained product molecular weight, indicating the synthesized product is our target. Calculation based on amino acid composition shows the theoretical molecular weight of the non-cyclized antimicrobial peptide is 1199.4; after cyclization, the molecular weight decreases by 18, becoming 1181.4, consistent with the obtained product molecular weight, indicating the synthesized product is the cyclized peptide form. During synthesis, the raw material for the amino acid at position 4 had its side chain protected by the ivdde protecting group, while the side chains of amino acids at other positions were protected by other protecting groups. Before cyclization, Hydrazine Hydrate/DMF was used to remove only the protecting group from the side chain of amino acid number 4, ensuring cyclization occurred between amino acids number 4 and number 10.

### 2. Antimicrobial Peptide Purity Analysis

Use 1 mg/mL antimicrobial peptide, with mobile phase A being 0.065% TFA in water (v/v), and mobile phase B being 0.05% TFA in acetonitrile (v/v). Perform separation and detection on a SHIMADZU HPLC-2030 instrument. Since the peak area is proportional to the amount of substance, the peak area percentage represents the purity of the antimicrobial peptide.

As shown in Figure 9, the peak area of the main substance accounts for 97.211% of the total product, meaning the purity of the main product is 97.211%. Based on the Mass Spectrum results which have already demonstrated the product is the target antimicrobial peptide, the purity of the target antimicrobial peptide is therefore 97.211%.

In summary, relying on its inherent high binding capacity to lipid A, the novel antimicrobial peptide provided herein possesses at least the following characteristics:
1. It inhibits the growth of common Gram-negative bacteria at low concentrations.
2. It inhibits the growth of polymyxin-resistant bacteria at low concentrations and demonstrates good efficacy.
3. The antimicrobial peptide has low cytotoxicity and nephrotoxicity.

Based on these characteristics, the novel antimicrobial peptide provided herein can serve as a new antibiotic, replacing polymyxins to function as the last line of defense.

## Claims

1. A compound of the following formula (1), a derivative thereof, a pharmaceutically acceptable salt thereof, or a solvate thereof:

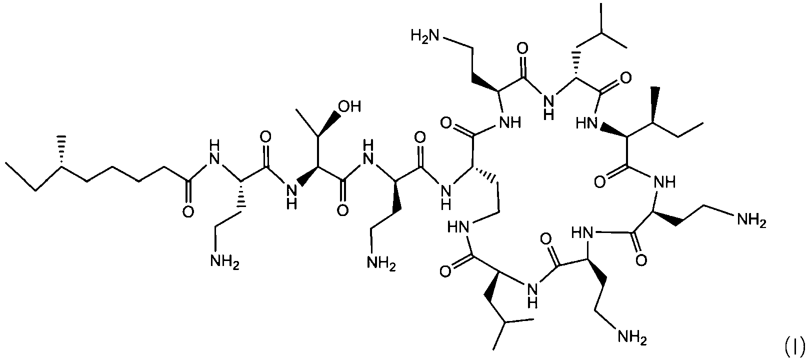

2. The compound, a derivative thereof, a pharmaceutically acceptable salt thereof, or a solvate thereof according to claim 1, wherein the salt is selected from the group consisting of hydrochloride, sulfate, phosphate, acetate, oxalate, succinate, fumarate, maleate, lactate, malate, tartrate, citrate, picrate, and any combination thereof.

3. The compound, a derivative thereof, a pharmaceutically acceptable salt thereof, or a solvate thereof according to claim 1 or 2, wherein the solvate is a hydrate or an ethanolate.

4. The compound, a derivative thereof, a pharmaceutically acceptable salt thereof, or a solvate thereof according to any one of claims 1-3, wherein the derivative is a PEG derivative or a polysaccharide derivative of the compound.

5. A pharmaceutical composition, comprising:
1) the compound, a derivative thereof, a pharmaceutically acceptable salt thereof, or a solvate thereof according to any one of claims 1-4; and
2) a pharmaceutically acceptable carrier or excipient.

6. The pharmaceutical composition according to claim 5, further comprising one or more antibiotics.

7. Use of the compound according to any one of claims 1-4, a derivative thereof, a pharmaceutically acceptable salt or solvate thereof, in the manufacture of a medicament for preventing or treating diseases associated with bacterial infections.

8. The use according to claim 7, wherein the bacteria are Gram-negative bacteria.

9. The use according to claim 7 or 8, wherein the bacteria are resistant to any polymyxin or antibiotic.

10. The use according to any one of claims 7-9, wherein the bacteria carry the mcr-1 gene.

11. The use according to any one of claims 7-10, wherein the bacteria are selected from the group consisting of *Escherichia coli, Acinetobacter baumannii, Pseudomonas aeruginosa,* and *Klebsiella pneumoniae.*

12. The use according to any one of claims 7-11, wherein the disease related to bacterial infection is selected from the group consisting of respiratory tract infection, pneumonia, bronchitis, wound infection, sepsis, and septicemia.

13. A method for treating a disease related to bacterial infection in a subject, comprising administering a therapeutically effective amount of the compound, a derivative thereof, a pharmaceutically acceptable salt thereof, or a solvate thereof according to any one of claims 1-4, or the pharmaceutical composition according to claim 5 or 6, to the subject.

14. The method according to claim 13, wherein the bacteria are Gram-negative bacteria.

15. The method according to claim 13 or 14, wherein the bacteria are resistant to any polymyxin or antibiotic.

16. The method according to any one of claims 13-15, wherein the bacteria carry the mcr-1 gene.

17. The method according to any one of claims 13-16, wherein the bacteria are selected from the group consisting of *Escherichia coli, Acinetobacter baumannii, Pseudomonas aeruginosa,* and *Klebsiella pneumoniae.*

18. The method according to any one of claims 13-17, wherein the disease related to bacterial infection is selected from the group consisting of respiratory tract infection, pneumonia, bronchitis, wound infection, sepsis, and septicemia.
